# EUROPEAN PATENT APPLICATION

(11) **EP 4 202 039 A1**
(43) Date of publication of application: **28.06.2023**
(21) Application number: 21858404.3
(22) Date of filing: 20.08.2021
(51) Int. Cl.: C12N 5/071

(54) **PRODUCTION METHOD FOR THREE-DIMENSIONAL CELL STRUCTURE, AND THREE-DIMENSIONAL CELL STRUCTURE**

(30) Priority: 20.08.2020 JP 2020139260
(71) Applicant: TOPPAN INC., Tokyo 110-0016 (JP)
(72) Inventor: SUZUKI Mizuho, Tokyo 110-0016 (JP); KITANO Shiro, Tokyo 110-0016 (JP)
(74) Representative: Plasseraud IP
(86) International application number: PCT/JP2021/030606
(87) International publication number: WO 2022/039261

(57) **Abstract**

A method for producing a three-dimensional cell structure having a vascular network includes a step of obtaining a mixture of a cell population, a cationic substance, a polyelectrolyte, and an extracellular matrix component, the cell population comprising at least endothelial cells; a step of collecting, from the obtained mixture, at least one cell aggregate comprising the at least endothelial cells, the cationic substance, the polyelectrolyte, and the extracellular matrix component; and a step of culturing the collected at least one cell aggregate in a medium, wherein a concentration of the extracellular matrix component in the mixture is 1.0×10⁻⁸ mg/mL or more and less than 2.5×10⁻² mg/mL.

## Description

### [Technical Field]

The present invention relates to methods for producing a three-dimensional cell structure, and to three-dimensional cell structures.

The present application claims priority to Japanese Patent Application No. 2020-139260 filed August 20, 2020, the entire contents of which are incorporated herein by reference.

### [Background Art]

In recent years, not only in regenerative medicine, but also in drug assay systems that require an environment close to that of the living body, the superiority of using three-dimensional cell structures that are three-dimensionally organized over using cells grown on plates has been demonstrated, and various techniques have been developed to construct three-dimensional cell structures in vitro. For example, a method of forming cell clusters on a surface substrate to which cells cannot become attached, a method of forming cell clusters in droplets, and a method of accumulating cells on a permeable membrane have been developed.

In order to maintain the organization of cells, extracellular matrixes (ECMs) such as collagen produced by the living body itself are necessary for cell-to-cell binding and scaffold formation. Therefore, external addition of ECMs when artificially constructing cell tissues has been studied.

For example, PTL 1 discloses a method for culturing cells isolated by enzyme treatment or the like as a three-dimensional aggregate after bringing the cells into contact with a water-soluble polymer that is a typical ECM and has a cell-protecting effect, such as collagen. According to this method, a water-soluble polymer gel is formed around the cells during culture.

PTL 2 discloses a method for constructing a three-dimensional cell structure by producing cell sheets using a culture dish on which a temperature-responsive resin, poly(N-isopropylacrylamide) (PIPAAm), is immobilized on the surface, and laminating the produced cell sheets.

PTL 3 discloses a method for constructing a three-dimensional cell structure by repeating a step of forming a cell layer and a step of alternately bringing the formed cell layer into contact with a first substance-containing liquid and a second substance-containing liquid, to thereby continuously laminate cell layers via a nanoscale-thick ECM. Since this method does not require detachment of single-layered cell sheets or lamination of detached cell sheets, it is said that three-dimensional cell structures can be produced with excellent reproducibility and efficiency.

PTL 4 discloses a method for constructing a three-dimensional cell structure by producing coated cells in which the entire surface of individual cells is coated with an adhesion membrane, and bonding the cells together through the adhesion membrane.

As described above, many methods have been proposed as techniques for producing three-dimensional cell structures. However, even if a three-dimensional cell structure has been temporarily formed by either method, unless a vascular network is reproduced inside the three-dimensional cell structure, it cannot be said that a structure actually close to that of the living body has been mimicked. In particular, in the case of a three-dimensional cell structure, it is required that a vascular network be well formed to supply necessary nutrients to the cells inside the three-dimensional cell structure.

In general, to form a vascular network, vascular endothelial cells are added to a cell population that constitutes a three-dimensional cell structure. However, a vascular network (also referred to as a blood vessel network) is not always formed only because the three-dimensional cell structure simply contains vascular endothelial cells. The surrounding environment of vascular endothelial cells is important for formation of a blood vessel network.

Various growth factors, such as VEGF, are known to affect the formation of blood vessel networks inside three-dimensional cell structures. As one of them, it has been reported that the mechanical properties of ECM affect the formation of blood vessel networks (see NPL 1).

The inventors of the present application have previously developed techniques for producing a three-dimensional cell structure, comprising a step A of obtaining a mixture in which cells are suspended in a solution comprising at least a cationic buffer solution, ECM, and a polyelectrolyte; step B of collecting the cells from the obtained mixture to form a cell aggregate on a substrate; and step C of culturing the cells to obtain a three-dimensional cell structure (see PTL 5 and PTL 6).

### [Citation List]

### [Patent Literature]

PTL 1: JP 2824081 B
PTL 2: WO 2002/008387
PTL 3: JP 4919464 B
PTL 4: JP 5850419 B
PTL 5: JP 6427836 B
PTL 6: JP 6639634 B

### [Non-Patent Literature]

NPL 1: Rouwkema J. and Khademhosseini A., Vascularization and Angiogenesis in Tissue Engineering: Beyond Creating Static Networks, Trends Biotechnol, 34 (9), 733-745, 2016.

### [Summary of Invention]

### [Technical Problem]

The methods disclosed in PTL 5 and PTL 6 can be applied without being significantly limited by cell types, and are effective methods that are excellent in capability to form a vascular network when the cells contain endothelial cells, and that can also form a layered structure.

However, the present inventors found that according to the methods of PTL 5 and PTL 6, a vascular network may not be formed in the three-dimensional cell structure.

Accordingly, an object of the present invention is to provide a technique for producing a three-dimensional cell structure stably having a vascular network.

### [Solution to Problem]

The present invention includes the following aspects.
[1] A method for producing a three-dimensional cell structure having a vascular network, the method comprising:
   a first step of performing a process of:
      obtaining a mixture of a cell population, a cationic substance, a polyelectrolyte, and an extracellular matrix component, the cell population comprising at least endothelial cells; and
      collecting, from the obtained mixture, at least one cell aggregate comprising the cell population, the cationic substance, the polyelectrolyte, and the extracellular matrix component; and
   a second step of performing a culturing of the collected at least one cell aggregate in a medium,
   wherein a concentration of the extracellular matrix component in the mixture is 1.0×10⁻⁸ mg/mL or more and less than 2.5×10⁻² mg/mL.
[2] The production method according to [1], wherein:
   the first step repeatedly performs the process one or more times to collect cell aggregates as the at least one cell aggregate; and
   the second step performs the culturing of each of the collected one or more cell aggregates.
[3] The production method according to [1] or [2], wherein:
   the extracellular matrix component is selected from collagen, laminin, fibronectin, vitronectin, elastin, tenascin, entactin, fibrillin, proteoglycan, and combinations thereof.
[4] The production method according to any one of [1] to [3], wherein:
   the polyelectrolyte is selected from glycosaminoglycans, dextran sulfate, rhamnan sulfate, fucoidan, carrageenan, polystyrene sulfonic acid, polyacrylamido-2-methylpropanesulfonic acid, polyacrylic acid, and combinations thereof.
[5] The production method according to any one of [1] to [4], wherein:
   a concentration of the polyelectrolyte in the mixture is 1.0×10⁻⁸ mg/mL or more and less than 2.5×10⁻² mg/mL.
[6] The production method according to any one of [1] to [5], wherein:
   the collecting step of the process comprises:
   removing a liquid part from the obtained mixture to obtain at least one cell aggregate comprising the cell population, the cationic substance, the polyelectrolyte, and the extracellular matrix component;
   suspending the obtained at least one aggregate in a solution to obtain a suspension thereof; and
   collecting the suspended at least one cell aggregate from the obtained suspension.
[7] The production method according to any one of [1] to [6], wherein:
   the collecting step of the process comprises collecting the at least one cell aggregate in a container having a bottom wall and a side wall,
   the at least one cell aggregate being collected in the container such that the at least endothelial cells of the at least one cell aggregate include cells located per unit area of the bottom wall of the container, the cells located per unit area of the bottom wall of the container having a density of 1.5×10⁴ cells/mm² or more and 1.5×10⁵ cells/mm² or less.
[8] A three-dimensional cell structure having a vascular network, comprising:
   a cell population comprising at least endothelial cells;
   a cationic substance;
   a polyelectrolyte; and
   an extracellular matrix component,
   the extracellular matrix component being contained in the three-dimensional cell structure in an amount of 0.01 mass% or more and 30 mass% or less.
[9] The three-dimensional cell structure according to [8], wherein:
   the at least endothelial cells include cells located per unit area of a bottom surface of the three-dimensional cell structure, the cells located per unit area of the bottom surface having a density of 1.5×10⁴ cells/mm² or more and 1.5×10⁵ cells/mm² or less.
[10] The three-dimensional cell structure according to [8] or [9], which has a thickness of 5 µm or more and 200 µm or less.

### [Advantageous Effect of Invention]

The present invention can provide a technique for producing a three-dimensional cell structure stably having a vascular network.

### [Brief Description of Drawings]

Fig. 1 shows fluorescence micrographs of three-dimensional cell structures taken in Experimental Example 1 when the final concentration of collagen and the final concentration of heparin are each 0.2 mg/mL, 0.1 mg/mL, 0.05 mg/mL, 0.025 mg/mL, and 0.0125 mg/mL.
Fig. 2 shows fluorescence micrographs of three-dimensional cell structures taken in Experimental Example 1 when the final concentration of collagen and the final concentration of heparin are each 0.05 mg/mL, 1.0×10⁻² mg/mL, 2.0×10⁻³ mg/mL, 4.0×10⁻⁴ mg/mL, 8.0×10⁻⁵ mg/mL, 1.6×10⁻⁵ mg/mL, 3.2×10⁻⁶ mg/mL, 6.4×10⁻⁷ mg/mL, 1.3×10⁻⁷ mg/mL, and 2.6×10⁻⁸ mg/mL.
Fig. 3 shows fluorescence micrographs of three-dimensional cell structures taken in Experimental Example 2 after the elapse of 5 days and 8 days from the start of culture of mixtures 2-1 and 2-2.

### [Description of Embodiments]

In an embodiment, the present invention provides a method for producing a three-dimensional cell structure having a vascular network, the method comprising:
a first step of performing a process of:
   obtaining a mixture of a cell population, a cationic substance, a polyelectrolyte, and an extracellular matrix component, the cell population comprising at least endothelial cells; and
   collecting, from the obtained mixture, at least one cell aggregate comprising the cell population, the cationic substance, the polyelectrolyte, and the extracellular matrix component; and
a second step of performing a culturing of the collected at least one cell aggregate in a medium,
wherein a concentration of the extracellular matrix component in the mixture is 1.0×10⁻⁸ mg/mL or more and less than 2.5×10⁻² mg/mL.

The production method of the present embodiment can provide a technique for producing a three-dimensional cell structure stably having a vascular network.

Further, in the vascular network of the three-dimensional cell structure produced by the production method of the present embodiment, the total length of vascular channels that constitute the vascular network may be 5,000 µm/mm² or more, for example, 5,200 µm/mm² or more, or 5,400 µm/mm² or more. In the present specification, the total length of vascular channels that constitute the vascular network is measured in the following manner. Fluorescence micrographs of the three-dimensional cell structure are taken with a fluorescence microscope (e.g., high-content confocal imaging system Operetta CLS, available from PerkinElmer). Cell structure imaging software Harmony (registered trademark, available from PerkinElmer) is used to analyze images of at least a 4.26 mm × 4.26 mm area when the culture container is a 96-well plate, or images of at least a 6.5 mm × 6.5 mm area when the culture container is a 24-well plate. More specifically, the culture container is designated as a region of interest (ROI), or the three-dimensional cell structure is stained with DAPI (4',6-diamidino-2-phenylindole) and ROI is designated. After removing noise by image flattening, the threshold of fluorescence intensity of the vascular channel is set for each image. Regions where the fluorescence intensity equals or exceeds the corresponding threshold and the size is small (e.g., 5 µm² or less) are excluded as noise. Then, the total length of the regions where the fluorescence intensity equals or exceeds the corresponding threshold is measured, and the result is taken as the total length of the vascular network.

The form of the three-dimensional cell structure is not particularly limited, and may be, for example, a three-dimensional cell structure formed by culturing cells within a scaffold composed of a natural biopolymer, such as collagen, or a synthetic polymer, or may be a cell aggregate (also referred to as spheroid), or may be a sheet-like cell structure.

In the present specification, the term "three-dimensional cell structure" refers to a three-dimensional cell aggregate containing at least one type of cell. Examples of the form of the three-dimensional cell structure include, but are not limited to, living tissue models, such as skin, hair, bone, cartilage, tooth, cornea, blood vessel, lymphatic vessel, heart, liver, pancreas, nerve, and esophagus; and solid cancer models, such as stomach cancer, esophageal cancer, bowel cancer, colon cancer, rectal cancer, pancreatic cancer, breast cancer, ovarian cancer, prostate cancer, renal cell cancer, and liver cancer.

The production method of the present embodiment comprises step (A) of obtaining a mixture of a cell population, a cationic substance, an extracellular matrix component, and a polyelectrolyte, the cell population comprising at least endothelial cells; step (B) of collecting, from the obtained mixture, at least one cell aggregate comprising the cell population, the cationic substance, the polyelectrolyte, and the extracellular matrix component; and step (C) of performing a culturing of the collected at least one cell aggregate in a medium.

In step (A), the cell population comprises at least endothelial cells. The endothelial cells are preferably vascular endothelial cells. Examples of the origin of the cells include, but are not limited to, humans, monkeys, dogs, cats, rabbits, pigs, cows, mice, rats, and the like.

Cells other than endothelial cells are not particularly limited, and may be, for example, somatic cells originating from bone, muscle, internal organs, nerve, brain, bone, skin, blood, or the like, or may be germ cells. Moreover, the cells may be induced pluripotent stem cells (iPS cells) or embryonic stem cells (ES cells). Alternatively, cultured cells, such as primary cultured cells, subcultured cells, and cell lines may be used. The cell population may contain one type of cell or a plurality of types of cells.

Examples of cells include, but are not limited to, nerve cells, dendritic cells, immune cells, vascular endothelial cells, lymphatic endothelial cells, fibroblasts, cancer cells such as liver cancer cells, epithelial cells, cardiac muscle cells, liver cells, pancreatic islet cells, tissue stem cells, smooth muscle cells, and the like.

In step (A), a cell population comprising at least endothelial cells, a cationic substance, a polyelectrolyte, and an extracellular matrix component are mixed to obtain a mixture. Because the mixture contains an extracellular matrix component, a thick cell structure tends to be easily obtained. By reducing the concentration of the extracellular matrix component in the mixture, a cell structure having a vascular network is likely to be stably obtained.

As the cationic substance used in the present embodiment, any positively charged substance can be used, as long as it does not adversely affect the cell growth and the formation of cell aggregates. Examples of cationic substances include, but are not limited to, cationic buffer solutions, such as tris-hydrochloric acid buffer solution, tris-maleic acid buffer solution, bis-tris buffer solution, and HEPES; ethanolamine, diethanolamine, triethanolamine, polyvinylamine, polyallylamine, polylysine, polyhistidine, polyarginine, and the like. Of these, cationic buffer solutions are preferable, and tris-hydrochloric acid buffer solution is more preferable.

The concentration of the cationic substance in step (A) is not particularly limited, as long as it does not adversely affect the cell growth and the formation of cell aggregates. The concentration of the cationic substance used in the present embodiment is preferably 10 to 100 mM, and may be, for example, 20 to 90 mM, 30 to 80 mM, 40 to 70 mM, or 45 to 60 mM.

When a cationic buffer solution is used as the cationic substance, the pH of the cationic buffer solution is not particularly limited, as long as it does not adversely affect the cell growth and the formation of cell aggregates. The pH of the cationic buffer solution used in the present embodiment is preferably 6.0 to 8.0. For example, the pH of the cationic buffer solution used in the present embodiment may be 7.0, 7.1, 7.2, 7.3, 7.4, 7.5, 7.6, 7.7, 7.8, 7.9, or 8.0. The pH of the cationic buffer solution used in the present embodiment is more preferably 7.2 to 7.6, and even more preferably 7.4.

As the extracellular matrix component used in the present embodiment, any component that constitutes an extracellular matrix (ECM) can be used, as long as it does not adversely affect the cell growth and the formation of cell aggregates. Examples of the extracellular matrix component include, but are not limited to, collagen, laminin, fibronectin, vitronectin, elastin, tenascin, entactin, fibrillin, proteoglycan, and the like. These extracellular matrix components may be used singly or in combination of two or more. Examples of the proteoglycan include, but are not limited to, chondroitin sulfate proteoglycan, heparan sulfate proteoglycan, keratan sulfate proteoglycan, and dermatan sulfate proteoglycan. The extracellular matrix component used in the present embodiment is preferably collagen, laminin, or fibronectin; collagen is particularly preferable. Modified forms and variants of the extracellular matrix components mentioned above may also be used, as long as they do not adversely affect the cell growth and the formation of cell aggregates.

The concentration of the extracellular matrix component used in the present embodiment is 1.0×10⁻⁸ mg/mL or more and less than 2.5×10⁻² mg/mL. The concentration of the extracellular matrix component used in the present embodiment is preferably 2.6×10⁻⁸ mg/mL or more and 1×10⁻² mg/mL or less, and may be, for example, 1.25×10⁻² mg/mL, 1×10⁻² mg/mL, 2.0×10⁻³ mg/mL, 4.0×10⁻⁴ mg/mL, 8.0×10⁻⁵ mg/mL, 1.6×10⁻⁵ mg/mL, 3.2×10⁻⁶ mg/mL, 6.4×10⁻⁷ mg/mL, 1.3×10⁻⁷ mg/mL, or 2.6×10⁻⁸ mg/mL. Because the concentration of the extracellular matrix component used in the present embodiment is 1.0×10⁻⁸ mg/mL or more and less than 2.5×10⁻² mg/mL, a three-dimensional cell structure stably having a vascular network can be produced. In the present embodiment, the extracellular matrix component may be dissolved in an appropriate solvent before use. Examples of solvents include, but are not limited to, water, buffer solutions, acetic acid, culture media, and the like. In the present embodiment, the extracellular matrix component is preferably dissolved in a medium or a buffer solution. Examples of media include, but are not limited to, basal media such as DMEM, E-MEM, MEMα, RPMI-1640, Mccoy'5a, and Ham's F-12; and media obtained by adding at least one serum of CS (bovine serum), FBS (fetal bovine serum), HBS (fetal horse serum), and the like to these basal media such that the at least one serum constitutes about 1 to 20 volume% of the media. Examples of buffer solutions include tris-hydrochloric acid buffer solution, tris-maleic acid buffer solution, bis-tris buffer solution, and HEPES.

In the present specification, the term "polyelectrolyte" refers to a polymer with a dissociable functional group in the polymer chain. As the polyelectrolyte used in the present embodiment, any polyelectrolyte can be used, as long as it does not adversely affect the cell growth and the formation of cell aggregates. Examples of polyelectrolytes include, but are not limited to, glycosaminoglycans such as heparin, chondroitin sulfate (e.g., chondroitin 4-sulfate and chondroitin 6-sulfate), heparan sulfate, dermatan sulfate, keratan sulfate, and hyaluronic acid; dextran sulfate, rhamnan sulfate, fucoidan, carrageenan, polystyrene sulfonic acid, polyacrylamido-2-methylpropanesulfonic acid, polyacrylic acid, and the like. These polyelectrolytes may be used singly or in combination of two or more. The polyelectrolyte used in the present embodiment is preferably a glycosaminoglycan. The polyelectrolyte used in the present embodiment is more preferably heparin, dextran sulfate, chondroitin sulfate, or dermatan sulfate. The polyelectrolyte used in the present embodiment is more preferably heparin. Derivatives of the above polyelectrolytes may also be used, as long as they do not adversely affect the cell growth and the formation of cell aggregates.

The concentration of the polyelectrolyte is not particularly limited, as long as it does not adversely affect the cell growth and the formation of cell aggregates. The concentration of the polyelectrolyte used in the present embodiment is preferably more than 0 mg/mL and less than 1.0 mg/mL. The concentration of the polyelectrolyte used in the present embodiment is preferably 1.0×10⁻⁸ mg/mL or more and less than 2.5×10⁻² mg/mL. The concentration of the extracellular matrix component used in the present embodiment is more preferably 2.6×10⁻⁸ mg/mL or more and 1×10⁻² mg/mL or less, and may be, for example, 1×10⁻² mg/mL, 2.0×10⁻³ mg/mL, 4.0×10⁻⁴ mg/mL, 1.6×10⁻⁵ mg/mL, 3.2×10⁻⁶ mg/mL, 1.3×10⁻⁷ mg/mL, or 2.6×10⁻⁸ mg/mL. Because the concentration of the extracellular matrix component used in the present embodiment is 1.0×10⁻⁸ mg/mL or more and less than 2.5×10⁻² mg/mL, a three-dimensional cell structure more stably having a vascular network can be produced. In the present embodiment, the polyelectrolyte may be dissolved in an appropriate solvent before use. Examples of solvents include, but are not limited to, water, buffer solutions, and culture media. When a cationic buffer solution is used as the cationic substance, the polyelectrolyte may be dissolved in the cationic buffer solution or a medium before use. Examples of cationic buffer solutions and media include buffer solutions and media that can be used to dissolve the extracellular matrix component.

An aspect of the present invention is the use of a polyelectrolyte in the method for producing a three-dimensional cell structure having a vascular network. The method for producing the three-dimensional cell structure is as described above. The polyelectrolyte may be any of the above polyelectrolytes, but is preferably heparin. The use of a polyelectrolyte in the method for producing a three-dimensional cell structure can prevent the formation of a vascular network from being inhibited even if the concentration of the extracellular matrix component is low as described above.

In the present embodiment, the mixing ratio between the polyelectrolyte and the extracellular matrix component is preferably 1:2 to 2:1. The mixing ratio between the polyelectrolyte and the extracellular matrix component used in the present embodiment is more preferably 1:1.5 to 1.5:1. The mixing ratio between the polyelectrolyte and the extracellular matrix component used in the present embodiment is even more preferably 1: 1.

In step (A), the cell population comprising at least endothelial cells, the cationic substance, the polyelectrolyte, and the extracellular matrix component can be mixed in a suitable container, such as a dish, tube, flask, bottle, or plate.

Subsequently, in step (B), one or more cell aggregates comprising the cell population, the cationic substance, the polyelectrolyte, and the extracellular matrix component are collected from the mixture obtained in step (A). In the present specification, the term "cell aggregate" refers to a population of cells. The cell aggregates also include cell precipitates obtained by centrifugation, filtration, or the like. In an embodiment, the cell aggregates are in the form of a slurry-like viscous body. The term "slurry-like viscous body" refers to a gel-like cell aggregate as described in Akihiro Nishiguchi et al., Cell-cell crosslinking by bio-molecular recognition of heparin-based layer-by-layer nanofilms, Macromol Biosci., 15 (3), 312-317, 2015.

As a means for collecting the cells in step (B), methods known to a person skilled in the art can be used. For example, the cells may be collected by centrifugation, magnetic separation, or filtration. The centrifugation conditions are not particularly limited, as long as they do not adversely affect the cell growth. For example, the mixture obtained in step (A) is seeded in a cell culture insert and centrifuged at 10°C and 400×g for 1 minute to collect the cells. Alternatively, the cells may be collected by spontaneous precipitation.

Further, step (B) may be step (B') of removing a liquid part from the mixture obtained in step (A), suspending the obtained at least one cell aggregate in a solution, and collecting the at least one cell aggregate from the obtained suspension. In step (B'), as a means for removing the liquid part from the mixture obtained in step (A) to collect at least one cell aggregate, methods known to a person skilled in the art can be used. For example, the liquid part may be removed by centrifugation or filtration. The centrifugation conditions are not particularly limited, as long as they do not adversely affect the cell growth and the formation of cell aggregates. For example, a microtube containing the mixture is subjected to centrifugation at room temperature and 400 to 1,000×g for 1 minute to separate a liquid part and at least one cell aggregate, thereby removing the liquid part. Alternatively, the liquid part may be removed after the cells are collected by spontaneous precipitation. In step (B'), as a means for collecting the at least one cell aggregate from the suspension obtained by suspending the at least one cell aggregate in a solution, methods known to a person skilled in the art can be used. For example, the liquid part can be removed from the suspension by centrifugation or filtration to thereby obtain the at least one cell aggregate in the container. Alternatively, the at least one cell aggregate may be obtained by spontaneous precipitation in the container. The at least one cell aggregate in step (B) or (B') may be in the form of laminae.

As for the number of cells collected in step (B), when, for example, at least one cell aggregate is obtained in a container having a bottom wall and a side wall, the number of cells present per unit area of the bottom wall is preferably 1.5×10⁴ cells/mm² or more and 1.5×10⁵ cells/mm² or less, for example, 3.0×10⁴ cells/mm² or more and 1.0×10⁵ cells/mm² or less, 4.0×10⁴ cells/mm² or more and 1.5×10⁵ cells/mm² or less, 5.0×10⁴ cells/mm² or more and 1.3×10⁵ cells/mm² or less, 6.1×10⁴ cells/mm² or more and 1.3×10⁵ cells/mm² or less, 7.5×10⁴ cells/mm² or more and 1.0×10⁵ cells/mm² or less, or 7.5×10⁴ cells/mm² or more and 9.6×10⁴ cells/mm² or less.

Subsequently, in step (C), the at least one cell aggregate is cultured in a medium to obtain a three-dimensional cell structure. The cell aggregate may be suspended in a solution before culture. The solution is not particularly limited, as long as it does not adversely affect the cell growth and the formation of a three-dimensional cell structure. For example, cell culture media, buffer solutions, and the like suitable for cells that constitute cell aggregates can be used, and cell culture media are preferable. Examples of cell culture media and buffer solutions include media and buffer solutions that can be used to dissolve the extracellular matrix component.

The cell aggregates can be suspended in a suitable container, such as a dish, tube, flask, bottle, or plate.

When the cell aggregate is suspended in a solution, the cells may be precipitated before culture to form cell precipitates on a substrate. Examples of substrates include culture containers for use in cell culture. The culture container may be a container of material and shape which are commonly used for culturing cells and microorganisms. Examples of the material of the culture container include, but are not limited to, glass, stainless steel, plastic, and the like. Examples of the culture container include, but are not limited to, dishes, tubes, flasks, bottles, plates, and the like. The substrate may be, for example, a material that allows liquid to pass through without allowing cells in the liquid to pass through. The substrate is preferably a permeable membrane. Examples of containers having a permeable membrane include, but are not limited to, cell culture inserts, such as Transwell (registered trademark) insert, Netwell insert, Falcon (registered trademark) cell culture insert, and Millicell (registered trademark) cell culture insert.

For cell precipitation, methods known to a person skilled in the art can be used. For example, the cells may be collected by centrifugation, magnetic separation, filtration, or the like. The centrifugation conditions are not particularly limited, as long as they do not adversely affect the cell growth. For example, the cells may be collected by seeding the mixture or suspension in a cell culture insert, followed by centrifugation at 10°C and 400×g for 1 minute. Alternatively, the cells may be collected by spontaneous precipitation. Further, the collected cells may form a layer structure.

Either the cell aggregate or the suspended cells when the cell aggregate is suspended are preferably seeded at a cell density of 1,000 cells/mm² or more, for example, 10,000 cells/mm² or more, 20,000 cells/mm² or more, or 25,000 cells/mm² or more. Further, the cells can be seeded at a cell density of, for example, 1,000,000 cells/mm² or less, 500,000 cells/mm² or less, 200,000 cells/mm² or less, or 100,000 cells/mm² or less. A more homogeneous three-dimensional cell structure can be obtained by performing a step of suspending at least one cell aggregate in a solution and a step of precipitating cells.

In step (C), the cells can be cultured under culture conditions suitable for the cells to be cultured. A person skilled in the art can select a suitable medium depending on the cell type and desired function. Examples of cell culture media include, but are not limited to, basal media such as DMEM, E-MEM, MEMα, RPMI-1640, Mccoy'5a, and Ham's F-12; and media obtained by adding serum, such as CS (bovine serum), FBS (fetal bovine serum), or HBS (fetal horse serum) to these basal media such that the serum constitutes about 1 to 20 volume% of the media. Conditions such as the temperature and atmospheric composition of the culture environment can also be easily determined by a person skilled in the art.

Substances for suppressing deformation of the constructed three-dimensional cell structure (e.g., contraction of tissue or detachment of tissue ends) may be added to the medium during cell culture. Examples of such substances include, but are not limited to, Y-27632, which is a selective ROCK (Rho-associated coiled-coil forming kinase/Rho binding kinase) inhibitor.

Step (C) is preferably performed after step (A) and step (B) or (B') are performed at least once. By repeating step (A) and step (B) or (B') before step (C), cell aggregates or cell precipitates can be laminated. This makes it possible to construct a three-dimensional cell structure with a plurality of layers. In this case, a plurality of types of cells may be used to construct a three-dimensional cell structure composed of different types of cells.

The three-dimensional cell structure produced by the production method of the present embodiment has a vascular network, and is thus useful as a model that mimics a structure close to that of the living body. In the present specification, the term "vascular network" refers to a network tubular structure with many branching points, such as a blood vessel network or lymphatic network, in living tissue.

The production method of the present embodiment enables production of a three-dimensional cell structure stably having a vascular network, and a three-dimensional cell structure having a sufficient thickness.

The thickness of the three-dimensional cell structure produced by the production method of the present embodiment may be 5 µm or more and 200 µm or less, and may be, for example, 10 µm or more and 150 µm or less, 20 µm or more and 100 µm or less, 20 µm or more and 50 µm or less, or 25 µm or more and 45 µm or less. By repeating steps (A) and (B) or steps (A) and (B') of the above method of the present invention, a three-dimensional cell structure stably having a vascular network can be produced while keeping a thickness of 5 to 100 µm, for example, in spite of a small amount of the extracellular matrix component. In the present embodiment, a section image of the cross section of the three-dimensional cell structure in the thickness direction is observed at 100 to 200x magnification.

The three-dimensional cell structure according to an embodiment of the present invention comprises a cell population comprising at least endothelial cells, a cationic substance, a polyelectrolyte, and an extracellular matrix component, and has a vascular network, and the extracellular matrix component is contained in the three-dimensional cell structure in an amount of 0.01 mass% or more and 30 mass% or less. The three-dimensional cell structure of the present embodiment is produced by the production method of the present embodiment.

The content of the extracellular matrix component in the three-dimensional cell structure of the present embodiment is 0.01 mass% or more and 30 mass% or less, preferably 0.1 mass% or more and 30 mass% or less, for example, 1 mass% or more and 30 mass% or less, 5 mass% or more and 30 mass% or less, 10 mass% or more and 30 mass% or less, 20 mass% or more and 30 mass% or less, or 20 mass% or more and 25 mass% or less. With the content of the extracellular matrix component in the three-dimensional cell structure of the present embodiment being 0.01 mass% or more and 30 mass% or less, a vascular network can be stably formed in the three-dimensional cell structure, and the three-dimensional cell structure can have a sufficient thickness.

In the three-dimensional cell structure of the present embodiment, the above cells may be preferably present per unit area of the bottom surface such that 1.5×10⁴ cells/mm² or more and 1.5×10⁵ cells/mm² or less, for example, 3.0×10⁴ cells/mm² or more and 1.0×10⁵ cells/mm² or less, 4.0×10⁴ cells/mm² or more and 1.5×10⁵ cells/mm² or less, 5.0×10⁴ cells/mm² or more and 1.3×10⁵ cells/mm² or less, 6.1×10⁴ cells/mm² or more and 1.3×10⁵ cells/mm² or less, 7.5×10⁴ cells/mm² or more and 1.0×10⁵ cells/mm² or less, or 7.5×10⁴ cells/mm² or more and 9.6×10⁴ cells/mm² or less.

The thickness of the three-dimensional cell structure of the present embodiment may be 5 µm or more and 200 µm or less, and may be, for example, 10 µm or more and 150 µm or less, 20 µm or more and 100 µm or less, 20 µm or more and 50 µm or less, or 25 µm or more and 45 µm or less.

The three-dimensional cell structure of the present embodiment stably has a vascular network and a sufficient thickness, and is thus useful as a model that mimics a structure close to that of the living body.

The present invention includes other aspects as follows.
[1] A method for producing a three-dimensional cell structure having a vascular network, the method comprising:
   a first step of performing a process of:
      obtaining a mixture of a cell population, a cationic substance, a polyelectrolyte, and an extracellular matrix component, the cell population comprising at least endothelial cells; and
      collecting, from the obtained mixture, at least one cell aggregate comprising the cell population, the cationic substance, the polyelectrolyte, and the extracellular matrix component; and
   a second step of performing a culturing of the collected at least one cell aggregate in a medium,
   wherein the extracellular matrix component is selected from collagen, laminin, fibronectin, and combinations thereof, the polyelectrolyte is a glycosaminoglycan, and the concentration of the extracellular matrix component in the mixture is 1.0×10⁻⁸ mg/mL or more and less than 2.5×10⁻² mg/mL.
[2] The production method according to [1], wherein:
   the first step repeatedly performs the process one or more times to collect cell aggregates as the at least one cell aggregate; and
   the second step performs the culturing of each of the collected one or more cell aggregates.
[3] The production method according to [1] or [2], wherein:
   the extracellular matrix component is collagen.
[4] The production method according to any one of [1] to [3], wherein:
   the polyelectrolyte is heparin.
[5] The production method according to any one of [1] to [4], wherein:
   a concentration of the polyelectrolyte in the mixture is 1.0×10⁻⁸ mg/mL or more and less than 2.5×10⁻² mg/mL.
[6] The production method according to any one of [1] to [5], wherein:
   the collecting step of the process comprises:
   removing a liquid part from the obtained mixture to obtain at least one cell aggregate comprising the cell population, the cationic substance, the polyelectrolyte, and the extracellular matrix component;
   suspending the obtained at least one aggregate in a solution to obtain a suspension thereof; and
   collecting the suspended at least one cell aggregate from the obtained suspension.
[7] The production method according to any one of [1] to [6], wherein:
   the collecting step of the process comprises collecting the at least one cell aggregate in a container having a bottom wall and a side wall,
   the at least one cell aggregate being collected in the container such that the at least endothelial cells of the at least one cell aggregate include cells located per unit area of the bottom wall of the container, the cells located per unit area of the bottom wall of the container having a density of 1.5×10⁴ cells/mm² or more and 1.5×10⁵ cells/mm² or less.
[8] A three-dimensional cell structure comprising:
   a cell population comprising at least endothelial cells;
   a cationic substance;
   a polyelectrolyte; and
   an extracellular matrix component,
   wherein:
      the extracellular matrix component is selected from collagen, laminin, fibronectin, and combinations thereof;
      the polyelectrolyte is a glycosaminoglycan; and
      the extracellular matrix component is contained in the three-dimensional cell structure in an amount of 0.01 mass% or more and 30 mass% or less.
[9] The three-dimensional cell structure according to [8], wherein:
   the at least endothelial cells include cells located per unit area of a bottom surface of the three-dimensional cell structure, the cells located per unit area of the bottom surface having a density of 1.5×10⁴ cells/mm² or more and 1.5×10⁵ cells/mm² or less.
[10] The three-dimensional cell structure according to [8] or [9], which has a thickness of 5 µm or more and 200 µm or less.
[11] The three-dimensional cell structure according to any one of [8] to [10], wherein:
   the extracellular matrix component is collagen.
[12] The production method according to any one of [8] to [11], wherein:
   the polyelectrolyte is heparin.
[13] Use of a polyelectrolyte in a method for producing the three-dimensional cell structure according to any one of [8] to [10].

### [Examples]

The present invention will be more specifically described in greater detail below by way of examples. However, the following examples should not limit the scope of the present invention.

In the following experimental examples, collagen I was used as collagen unless otherwise specified.

### [Experimental Example 1]

### (Three-dimensional cell structure formation 1)

1.38×10⁷ cells of normal human dermal fibroblasts (NHDF) and 2.1×10⁵ cells of GFP-labeled human umbilical vein endothelial cells (GFP-HUVEC) were suspended in a liquid mixture of 0.5 mL of 0.8 mg/mL, 0.4 mg/mL, or 0.2 mg/mL heparin/50 mM tris-hydrochloric acid buffer solution (pH: 7.4), 0.5 mL of collagen of the same concentration/50 mM tris-hydrochloric acid buffer solution (pH: 7.4), and 1 mL of 10% fetal bovine serum (FBS)-containing Dulbecco's modified eagle medium (DMEM medium). As a result, the final concentration of collagen and the final concentration of heparin were each 0.2 mg/mL, 0.1 mg/mL, and 0.05 mg/mL. Further, the liquid mixture in which the final concentration of collagen and the final concentration of heparin were each 0.05 mg/mL was gradually diluted 2-fold with DMEM medium to prepare a liquid mixture in which the final concentration of collagen and the final concentration of heparin were each 0.025 mg/mL or 1.25×10⁻² mg/mL. Then, the above normal human dermal fibroblasts (NHDF) and GFP-labeled human umbilical vein endothelial cells (GFP-HUVEC) were similarly suspended therein. Further, the liquid mixture in which the final concentration of collagen and the final concentration of heparin were each 0.05 mg/mL was gradually diluted 5-fold with DMEM medium to prepare a liquid mixture in which the final concentration of collagen and the final concentration of heparin were each 1.0×10⁻² mg/mL, 2.0×10⁻³ mg/mL, 4.0×10⁻⁴ mg/mL, 8.0×10⁻⁵ mg/mL, 1.6×10⁻⁵ mg/mL, 3.2×10⁻⁶ mg/mL, 6.4×10⁻⁷ mg/mL, 1.3×10⁻⁷ mg/mL, or 2.6×10⁻⁸ mg/mL. Then, the above normal human dermal fibroblasts (NHDF) and GFP-labeled human umbilical vein endothelial cells (GFP-HUVEC) were similarly suspended therein.

Subsequently, the entire amount of the resulting mixture was seeded in a 96-well cell culture insert (the bottom area per well was 0.143 cm²), and centrifuged at room temperature and 400×g for 1 minute. In this way, a cell layer was formed on the cell culture insert. The number of cells seeded in the cell culture insert was 9.0×10⁵ cells for NHDF and 1.35×10⁴ cells for GFP-HUVEC.

Subsequently, the cells were cultured in a CO₂ incubator (37°C, 5% O₂) for 24 hours (the time at which this culture was started was defined as the culture start time). After culturing for 24 hours from the culture start time, the medium was replaced with 10% FBS-containing DMEM. Thereafter, the medium was replaced 48 hours and 120 hours after the culture start time, and the culture was continued for 8 days in total.

### <<Observation of three-dimensional cell structure>>

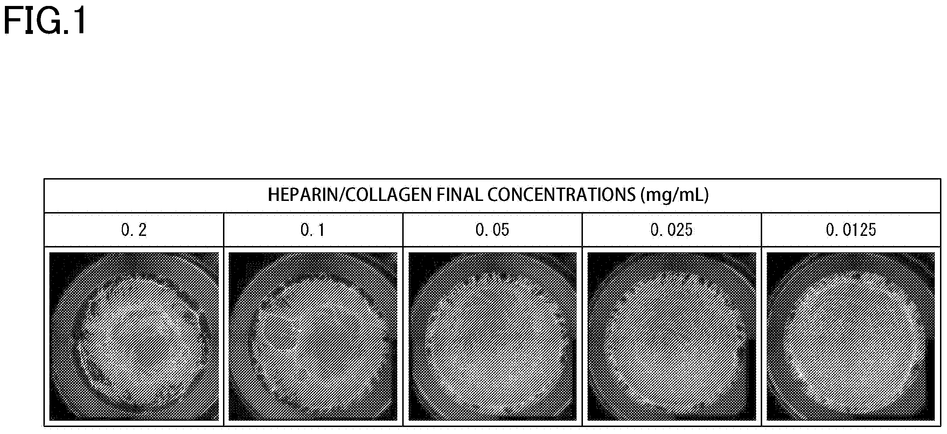

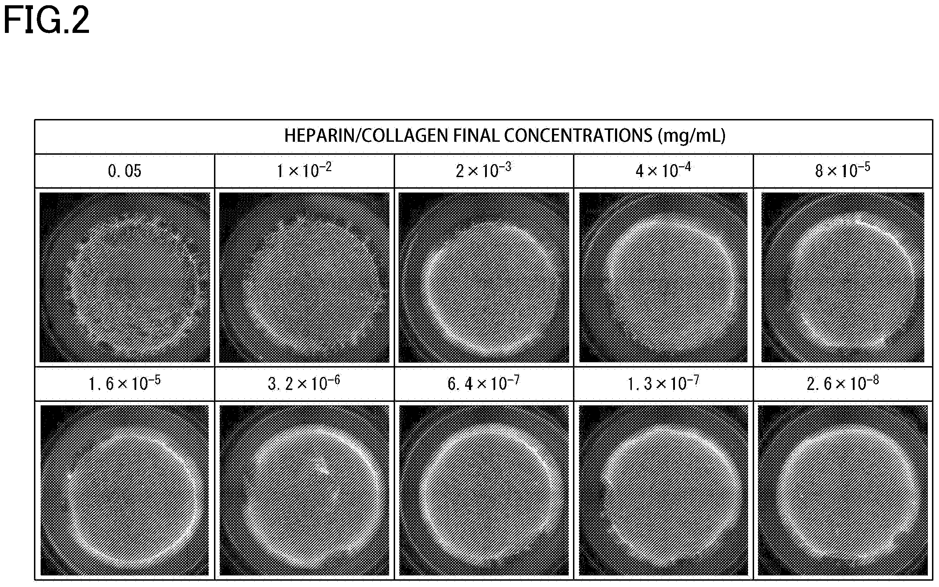

8 days after the culture start time, the three-dimensional cell structure was fixed in a 10% formalin solution, then vascular endothelial cells were fluorescently labeled with CD31 antibody (available from DAKO, JC70A M082329) and secondary antibody (available from Invitrogen, A-11001), and whether a blood vessel network structure was formed was observed using a fluorescence microscope (available from PerkinElmer, Operetta CLS). Fig. 1 shows photographs showing the results of fluorescence microscopic observation when the final concentration of collagen and the final concentration of heparin were each 0.2 mg/mL, 0.1 mg/mL, 0.05 mg/mL, 0.025 mg/mL, and 0.0125 mg/mL. Fig. 2 shows photographs showing the results of fluorescence microscopic observation when the final concentration of collagen and the final concentration of heparin were each 0.05 mg/mL, 1.0×10⁻² mg/mL, 2.0×10⁻³ mg/mL, 4.0×10⁻⁴ mg/mL, 8.0×10⁻⁵ mg/mL, 1.6×10⁻⁵ mg/mL, 3.2×10⁻⁶ mg/mL, 6.4×10⁻⁷ mg/mL, 1.3×10⁻⁷ mg/mL, and 2.6×10⁻⁸ mg/mL. In Figs. 1 and 2, the phrase "HEPARIN/COLLAGEN FINAL CONCENTRATIONS" indicates the final concentrations of heparin and collagen. From the results of microscopic observation in Figs. 1 and 2, whether a blood vessel network structure was formed was evaluated. Evaluation was carried out based on the following criteria. Table 1 shows the results of evaluation.
A blood vessel network structure was formed in the entire three-dimensional cell structure: A
A blood vessel network structure was not formed in part of the three-dimensional cell structure: B
A blood vessel network structure was not formed anywhere in the entire three-dimensional cell structure: C

**[Table 1]**

| Final concentration of collagen (mg/mL) | Final concentration of heparin (mg/mL) | Evaluation of formation of blood vessel network structure |
|---|---|---|
| 0.2 | 0.2 | B |
| 0.1 | 0.1 | B |
| 0.05 | 0.05 | B |
| 0.025 | 0.025 | B |
| 1.25×10⁻² | 1.25×10⁻² | A |
| 1.0×10⁻² | 1.0×10⁻² | A |
| 2.0×10⁻³ | 2.0×10⁻³ | A |
| 4.0×10⁻⁴ | 4.0×10⁻⁴ | A |
| 8.0×10⁻⁵ | 8.0×10⁻⁵ | A |
| 1.6×10⁻⁵ | 1.6×10⁻⁵ | A |
| 3.2×10⁻⁶ | 3.2×10⁻⁶ | A |
| 6.4×10⁻⁷ | 6.4×10⁻⁷ | A |
| 1.3×10⁻⁷ | 1.3×10⁻⁷ | A |
| 2.6×10⁻⁸ | 2.6×10⁻⁸ | A |

As shown in Table 1, it was found that when the final concentration of heparin and the final concentration of collagen were 2.6×10⁻⁸ mg/mL to 1.25×10⁻² mg/mL, a blood vessel network structure was formed in the entire three-dimensional cell structure. It was found that when the final concentration of heparin and the final concentration of collagen were 0.025 to 0.2 mg/mL, a blood vessel network structure was not formed in part of the three-dimensional cell structure in some cases. Further, it was revealed that a blood vessel network structure tended to be more easily formed when the heparin concentration and the collagen concentration were lower.

### <<Measurement of thickness of three-dimensional cell structured

Subsequently, the thickness of each three-dimensional cell structure was measured. The thickness was calculated using ImageJ for a total of three points, one point in the central portion of the section image and two points at both ends, and the arithmetic mean value was taken as the thickness of the three-dimensional cell structure. Table 2 shows the results of measuring the thickness of each three-dimensional cell structure.

**[Table 2]**

| Final concentration of collagen (mg/mL) | Final concentration of heparin (mg/mL) | Thickness of three-dimensional cell structure (µm) |
|---|---|---|
| 0.05 | 0.05 | 91.5 |
| 1.0×10⁻² | 1.0×10⁻² | 44.7 |
| 2.0×10⁻³ | 2.0×10⁻³ | 25.6 |
| 4.0×10⁻⁴ | 4.0×10⁻⁴ | 26.1 |
| 8.0×10⁻⁵ | 8.0×10⁻⁵ | 27.1 |
| 1.6×10⁻⁵ | 1.6×10⁻⁵ | 37.2 |
| 3.2×10⁻⁶ | 3.2×10⁻⁶ | 25.2 |
| 6.4×10⁻⁷ | 6.4×10⁻⁷ | 25.2 |
| 1.3×10⁻⁷ | 1.3×10⁻⁷ | 24.1 |
| 2.6×10⁻⁸ | 2.6×10⁻⁸ | 25.6 |

The results revealed that when the final concentration of collagen was 2.6×10⁻⁸ mg/mL or more and the final concentration of heparin was 2.6×10⁻⁸ mg/mL or more, the three-dimensional cell structure had a thickness of more than 20 µm.

### [Experimental Example 2]

### (Three-dimensional cell structure formation 2)

A liquid mixture was prepared using 0.5 mL of 0.2 mg/mL heparin/50 mM tris-hydrochloric acid buffer solution (pH: 7.4), 0.5 mL of 0.2 mg/mL collagen/50 mM tris-hydrochloric acid buffer solution (pH: 7.4), and 1 mL of 10% fetal bovine serum (FBS)-containing Dulbecco's modified eagle medium (DMEM medium). The liquid mixture was further diluted 4-fold with DMEM medium to prepare a liquid mixture in which the final concentration of collagen and the final concentration of heparin were each 1.25×10⁻² mg/mL, and 1.38×10⁻⁷ cells of NHDF and 2.1×10⁵ cells of GFP-HUVEC were suspended therein to obtain a mixture 2-1.

In place of 0.5 mL of the 0.2 mg/mL heparin/50 mM tris-hydrochloric acid buffer solution (pH: 7.4), 0.5 mL of 50 mM tris-hydrochloric acid buffer solution (pH: 7.4) was added to prepare a liquid mixture in which the final concentration of collagen and the final concentration of heparin were 1.25×10⁻² mg/mL and 0 mg/mL, respectively. Other than the above, the same procedure was performed to obtain a mixture 2-2.

Subsequently, the entire amount of each of the resulting mixtures 2-1 and 2-1 was seeded in a 96-well cell culture insert (the bottom area per well was 0.143 cm²), and centrifuged at room temperature and 400×g for 1 minute. In this way, a cell layer was formed on the cell culture insert. The number of cells seeded in the cell culture insert was 9.0×10⁵ cells for NHDF and 1.35×10⁴ cells for GFP-HUVEC.

Subsequently, the cells were cultured in a CO₂ incubator (37°C, 5% O₂) for 24 hours (the time at which this culture was started was defined as the culture start time). After culturing for 24 hours from the culture start time, the medium was replaced with 10% FBS-containing DMEM. Thereafter, the medium was replaced 48 hours and 120 hours after the culture start time, and the culture was continued for 8 days in total.

8 days after the culture start time, whether a blood vessel network structure was formed was observed in the same manner as in <<Observation of three-dimensional cell structure>>, and the stained area of the blood vessel network was calculated by the following method. Fluorescence micrographs of the three-dimensional cell structure were taken with a fluorescence microscope (available from PerkinElmer, high-content confocal imaging system Operetta CLS), and images of a 4.26 mm × 4.26 mm area were analyzed using cell structure imaging software Harmony (registered trademark, produced by PerkinElmer). More specifically, the cell culture insert was designated as ROI. After removing noise by image flattening, the threshold of fluorescence intensity of the vascular channel was set for each image. Regions where the fluorescence intensity equaled or exceeded the corresponding threshold and the size was small (5 µm² or less) were excluded as noise. Then, the total area of the regions where the fluorescence intensity equaled or exceeded the corresponding threshold was measured, and the result was taken as the stained area of the vascular network.

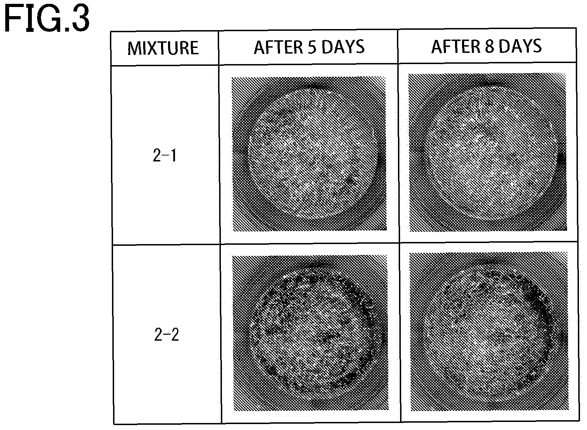

Table 3 shows the results of evaluating the stained area of the blood vessel network and the formation of a blood vessel network structure 5 days and 8 days after the start of culture of the mixtures 2-1 and 2-2. The stained area of the blood vessel network is indicated as a relative ratio when the stained area of the blood vessel network 8 days after the start of culture of the mixture 2-1 is taken as 100%. Further, Fig. 3 shows the results of fluorescence microscopic observation of the three-dimensional cell structures 5 days and 8 days after the start of culture of the mixtures 2-1 and 2-2.

**[Table 3]**

| Mixture | Final concentration of collagen (mg/mL) | Final concentration of heparin (mg/mL) | Stained area of blood vessel network (%) | | Evaluation of formation of blood vessel network structure | |
|---|---|---|---|---|---|---|
| | | | After 5 days | After 8 days | After 5 days | After 8 days |
| 2-1 | 1.25×10⁻² | 1.25×10⁻² | 107.6 | 100 | A | A |
| 2-2 | 1.25×10⁻² | 0 | 87.7 | 88.5 | B | B |

As shown in Table 3 and Fig. 3, it was found that even when the final concentration of collagen was 1.25×10⁻² mg/mL, if heparin was not contained, a blood vessel network structure was not formed in part of the three-dimensional cell structure in some cases.

### [Industrial Applicability]

The present invention can provide a technique for producing a three-dimensional cell structure stably having a vascular network.

## Claims

1. A method for producing a three-dimensional cell structure having a vascular network, the method comprising:
a first step of performing a process of:
obtaining a mixture of a cell population, a cationic substance, a polyelectrolyte, and an extracellular matrix component, the cell population comprising at least endothelial cells; and
collecting, from the obtained mixture, at least one cell aggregate comprising the cell population, the cationic substance, the polyelectrolyte, and the extracellular matrix component; and
a second step of performing a culturing of the collected at least one cell aggregate in a medium,
wherein a concentration of the extracellular matrix component in the mixture is 1.0×10⁻⁸ mg/mL or more and less than 2.5×10⁻² mg/mL.

2. The production method according to claim 1, wherein
the first step repeatedly performs the process one or more times to collect cell aggregates as the at least one cell aggregate; and
the second step performs the culturing of each of the collected one or more cell aggregates.

3. The production method according to claim 1 or 2, wherein:
the extracellular matrix component is selected from collagen, laminin, fibronectin, vitronectin, elastin, tenascin, entactin, fibrillin, proteoglycan, and combinations thereof.

4. The production method according to any one of claims 1 to 3, wherein:
the polyelectrolyte is selected from glycosaminoglycans, dextran sulfate, rhamnan sulfate, fucoidan, carrageenan, polystyrene sulfonic acid, polyacrylamido-2-methylpropanesulfonic acid, polyacrylic acid, and combinations thereof.

5. The production method according to any one of claims 1 to 4, wherein:
a concentration of the polyelectrolyte in the mixture is 1.0×10⁻⁸ mg/mL or more and less than 2.5×10⁻² mg/mL.

6. The production method according to any one of claims 1 to 5, wherein:
the collecting step of the process comprises:
removing a liquid part from the obtained mixture to obtain at least one cell aggregate comprising the cell population, the cationic substance, the polyelectrolyte, and the extracellular matrix component;
suspending the obtained at least one aggregate in a solution to obtain a suspension thereof; and
collecting the suspended at least one cell aggregate from the obtained suspension.

7. The production method according to any one of claims 1 to 6, wherein:
the collecting step of the process comprises collecting the at least one cell aggregate in a container having a bottom wall and a side wall,
the at least one cell aggregate being collected in the container such that the at least endothelial cells of the at least one cell aggregate include cells located per unit area of the bottom wall of the container, the cells located per unit area of the bottom wall of the container having a density of 1.5×10⁴ cells/mm² or more and 1.5×10⁵ cells/mm² or less.

8. A three-dimensional cell structure having a vascular network, comprising:
a cell population comprising at least endothelial cells;
a cationic substance;
a polyelectrolyte; and
an extracellular matrix component,
the extracellular matrix component being contained in the three-dimensional cell structure in an amount of 0.01 mass% or more and 30 mass% or less.

9. The three-dimensional cell structure according to claim 8, wherein:
the at least endothelial cells include cells located per unit area of a bottom surface of the three-dimensional cell structure, the cells located per unit area of the bottom surface having a density of 1.5×10⁴ cells/mm² or more and 1.5×10⁵ cells/mm² or less.

10. The three-dimensional cell structure according to claim 8 or 9, which has a thickness of 5 µm or more and 200 µm or less.
